(19) Europäisches Patentamt
European Patent Office
Office européen des brevets

(11)     EP 1 813 156 B1

(12)     EUROPEAN PATENT SPECIFICATION

(45) Date of publication and mention
of the grant of the patent:
**29.08.2012 Bulletin 2012/35**

(51) Int Cl.:
*A23L 2/52* (2006.01)          *A23L 1/30* (2006.01)
*A23L 2/38* (2006.01)          *A61K 31/353* (2006.01)
*A61K 33/14* (2006.01)

(21) Application number: **05807100.2**

(22) Date of filing: **15.11.2005**

(86) International application number:
**PCT/JP2005/020930**

(87) International publication number:
**WO 2006/051980 (18.05.2006 Gazette 2006/20)**

(54) **PACKED BEVERAGE FOR LIPID COMBUSTION PROMOTION**

VERPACKTES GETRÄNK ZUR FÖRDERUNG DER LIPIDVERBRENNUNG

BOISSON CONDITIONNÉE FAVORISANT LA COMBUSTION DES GRAISSES

(84) Designated Contracting States:
**DE ES FR GB IT**

(30) Priority: **15.11.2004  JP 2004331247**
**15.03.2005  JP 2005072832**

(43) Date of publication of application:
**01.08.2007  Bulletin 2007/31**

(73) Proprietor: **Kao Corporation**
**Chuo-ku**
**Tokyo 103-8210 (JP)**

(72) Inventors:
• **KATAOKA, Kiyoshi,**
**c/o Kao Corporation Research Lab**
**Tokyo 1318501 (JP)**
• **TAKASHIMA, Shinichiro,**
**c/o Kao Corporation Rch Lab**
**Tokyo 1318501 (JP)**
• **IWASAKI, Masaki,**
**c/o Kao Corporation Research Lab.**
**Tokyo 1318501 (JP)**
• **HOSHINO, Eiichi,**
**c/o Kao Corporation Research Lab.**
**Tokyo 1318501 (JP)**
• **SHIBATA, Eiichiro,**
**c/o Kao Corporation Rch. Lab.**
**Tokyo 1318501 (JP)**

(74) Representative: **HOFFMANN EITLE**
**Patent- und Rechtsanwälte**
**Arabellastrasse 4**
**81925 München (DE)**

(56) References cited:
EP-A- 1 695 628          EP-A- 1 695 630
EP-A1- 1 695 629          JP-A- 2002 326 932
JP-A- 2004 129 670          JP-A- 2004 159 634
JP-A- 2004 222 640          JP-A- 2005 058 208
JP-A- 2005 058 209          JP-A- 2005 058 210
JP-A- 2005 058 211          JP-A- 2005 160 367
JP-A- 2005 160 368          JP-A- 2005 176 606

• OTA N ET AL: "EFFECTS OF COMBINATION OF REGULAR EXERCISE AND TEA CATECHINS INTAKE ON ENERGY EXPENDITURE IN HUMANS" JOURNAL OF HEALTH SCIENCE - EISEI KAGAKU, PHARMACEUTICAL SOCIETY OF JAPAN, JP, vol. 51, no. 2, 1 January 2005 (2005-01-01), pages 233-236, XP008072864 ISSN: 1344-9702

**Description**

**Field of the Invention**

This invention relates to a packaged beverage for accelerating fat-burning. Specifically, this invention relates to a packaged beverage for accelerating fat-burning anda fat-burning accelerator, apackagedbeverage for boosting energy-consumption and an energy-consumption booster, and a packaged beverage for enhancing exercise endurance and an exercise-endurance enhancer, which are to be ingested preferably while exercising.

**Background of the Invention**

Fat is an important nutrient along with protein and carbohydrates, and is useful as an energy source, but its high calorie (9 kcal/g) is a primary cause of obesity and raises problems such as life-style related diseases. Fat-rich meals are delicious and modern people are increasingly getting accustomed to such meals, so fat and obesity have become a nationwide problem in addition to a rise in medical expenses, in developed countries in this opulent era. From such background, there is an increasing concern in recent years in the maintenance and promotion of health and also in the prevention and treatment of diseases, and therefore, a great deal of work is being conducted in order to elucidate the relationship between fat or obesity and life-style related diseases.

Conventional research is primarily concerned with fatty acids which make up triglycerides as principal components of fat, resulting in the development of oil and fat substitutes and unabsorbable oils and fats. Other research has also been conducted with a view to increasing the inherent metabolic function of the living body for fat and preventing obesity by ingesting a third ingredient. Known examples of such a third ingredient include oolong-tea polyphenols, capsaicin, hydroxycitric acid contained in garcinia, and the like. When given to high-fat-fed rats, oolong-tea polyphenols increase the fecal fat excretion and in addition, induces the activation of lipase, a lipolytic enzyme. Capsaicin acts on the brain to stimulate the excretion of adrenaline from the adrenal. On the other hand, hydroxycitric acid is considered to inhibit the synthesis of fat. Plant-derived ingredients such as capsaicin and caffeine have been reported to have an effect to promote the metabolism of fat and hence, the degradation of fat (Non-patent Document 1 and Non-patent Document 2), but no empirical research has been made as to their effect on humans at actual use levels. As a matter of fact, such sufficient effect remains to be seen at any practical level.

Further, catechins contained in green tea, black tea, oolong tea and the like are known to be effective for the suppression of an increase in cholesterol level (Patent Document 1), the inhibition of $\alpha$-amylase activity (Patent Document 2), and the accelerated burning of accumulated fats, the accelerated burning of dietary fat, the acceleration of expression of the $\beta$-oxidation gene in the liver, and the like (Patent Document 3).

However, the fat-burning effect indicated in Patent Document 3 is a finding that was obtained as a result of an observation of changes in breath composition with time from immediately after single ingestion of catechins. Concerning the body-fat inhibitory effect making use of catechins, there is, accordingly, a desire for a beverage having still better effect. For sports drinks to be consumed under exercise load or the like, there is an outstanding demand for further improvements in taste and also in body-fat inhibitory effect, energy-consumption increasing effect and exercise-endurance boosting effect.

Patent Document 1: JP-A-60-156614
Patent Document 2: JP-A-3-133928
Patent Document 3: JP-A-2002-326932
Non-patent Document 1: Yoshida, et al., J. Nutr. Sci. Vitaminol, 34(6), 587-594, 1988 Dec.
Non-patent Document: Yoshioka, et al., J. Nutr. Sci . Vitaminol, 36(2), 173-178, 1990 Apr.

**Disclosure of the Invention**

This invention is defined by the claims and relates to a packaged beverage for accelerating fat-burning with a green tea extract added therein, wherein the packaged beverage has a pH of from 2 to 7 and contains the following ingredients (A) to (C):

(A) from 0.01 to 1.0 wt% of non-polymer catechins,
(B) from 0.001 to 0.5 wt% of sodium ions, and
(C) from 0.001 to 0.2 wt% of potassium ions.

This invention also relates to the use of said packaged beverage. for boosting energy-consumption, for enhancing exercise-endurance, and for preventing exercise-induced fatigue, wherein it contains a green tea extract and has a pH of from 2 to 7, and further contains the following ingredients (A) to (C):

>(A) from 0.01 to 1.0 wt% of non-polymer catechins,
>(B) from 0.001 to 0.5 wt% of sodium ions, and
>(C) from 0.001 to 0.2 wt% of potassium ions.

This invention yet further relates to a method for boosting energy-consumption, a method for enhancing exercise-endurance and a method for preventing exercise-fatigue, which includes consuming a beverage, wherein the beverage contains a green tea extract added therein and has a pH of from 2 to 7, and further contains the following ingredients (A) to (C):

>(A) from 0.01 to 1.0 wt% of non-polymer catechins,
>(B) from 0.001 to 0.5 wt% of sodium ions, and
>(C) from 0.001 to 0.2 wt% of potassium ions.

**Brief Description of the Drawings**

FIG. 1 is a diagram showing a relationship between exercise loads (watt) and differences in fat oxidation (differences between week 0 and week 12).
FIG. 2 is a diagram illustrating a relationship between the extent of exercise habits and differences in body weight (differences between week 0 and week 12).

**Modes for Carrying out the Invention**

Catechin-containing beverages used to date are excellent in having a fat-burning acceleration effect. However, there is still a great demand for developing a material that is easier to ingest and has a high efficacy of fat-burning. The present invention, therefore, relates to a packaged beverage for accelerating fat-burning; fat-burning accelerator; packaged beverage for boosting energy-consumption;energy-consumption booster;beveragefor enhancing exercise-endurance; exercise-endurance enhancer; beverage for preventing exercise-induced fatigue; and preventive agent for exercise-fatigue. All of them are easy to ingest and are excellent in fat-burning acceleration effect.

Paying attention to the use of a body weight, body fat mass or the like as an index of body fat, the present inventors conducted an investigation by using a solution which contained non-polymer catechins at a high concentration owing to the addition of a green tea extract and also contained predetermined amounts of sodium ions and potassium ions. As a result, it has been found that, in a case that catechins were not ingested at all shortly before an observation was made for any changes in the composition of breath, exercising fat-burning effect increases relative to resting fat-burning effect despite the existence of no free catechins in blood, although catechins had been ingested over a long period by continued ingestion of the solution, and also that a combination of a long-term exercise habit and the ingestion of catechins can bring about a synergistic body-fat reducing effect. It has also been found that the ingestion of catechins leads to an increase in maximum oxygen uptake as an index of exercise endurance as a result of accelerated fat-burning and increased energy-consumption.

The ingestion of the packaged beverage for accelerating fat-burning or fat-burning accelerator according to the present invention accelerates fat-burning and facilitates the use of fat as energy, thereby reducing body fat significantly. These effects are synergistically enhanced when combined with exercise. In addition, the energy consumption is increased, the maximum oxygen uptake is increased, and the exercise endurance is enhanced.

The packaged beverage for accelerating fat-burning, fat-burning accelerator, packaged beverage for boosting energy-consumption, energy-consumption booster, packaged beverage for enhancing exercise-endurance, exercise-endurance enhancer, packaged beverage for preventing exercise-induced fatigue and preventive agent for exercise-induced fatigue relating to the present invention, which may hereinafter be collectively called "the packaged beverage for accelerating fat-burning and the like", are each a solution, which contains a green tea extract added therein, has a pH of from 2 to 7, and further contains the following ingredients (A) to (C):

>(A) from 0.01 to 1.0 wt% of non-polymer catechins,
>(B) from 0.001 to 0.5 wt% of sodium ions, and
>(C) from 0.001 to 0.2 wt% of potassium ions.

The term "non-polymer catechins (A) " as used herein is a generic term, which collectively encompasses non-epicatechins such as catechin, gallocatechin, catechingallate and gallocatechingallate, and epicatechins such as epicatechin, epigallocatechin, epicatechingallate and epigallocatechingallate.

The packaged beverage for accelerating fat-burning according to the present invention contains the non-polymer catechins (A), each of which is in a dissolved form in water, at a content of from 0.01 to 1.0 wt%, preferably from 0.03 to 0.5 wt. %, more preferably from 0.04 to 0.4 wt.%, even more preferably from 0.05 to 0.3 wt.%, even more preferably from 0. 06 to 0. 3 wt. %, even more preferably from 0.092 to 0.26 wt%, yet even more preferably from 0.1 to 0.15 wt%. Insofar as the content of non-polymer catechins falls within the above-described range, a great deal of non-polymer catechins can be consumed with ease, and from the standpoint of the color tone of the beverage shortly after its preparation, this content range is also preferred. The concentration of the non-polymer catechins can be adjusted by relying upon the amount of a green tea extract to be added.

The packaged beverage for accelerating fat-burning and the like according to the present invention each contains from 0.001 to 0.5 wt% of sodium ions (B) and from 0.001 to 0.2 wt% of potassium ions (C). Concentrations of sodium ions (B) and potassium ions (C) lower than 0.001 wt% cannot bring about the effect of enhancing the fat-burning acceleration of the non-polymer catechins. Depending on the scenario in which the packaged beverage is consumed, the packaged beverage may be unfulfilling in taste, and may fail to provide effective replenishment of minerals. Accordingly, such low concentrations are not preferred. When their concentrations exceed 0.5 wt%, on the other hand, the taste of, specifically ther salts themselves become strong, thereby making the packaged beverage unsuited for consuming over a long term. Moreover, the color tone significantly varies at such high concentrations when stored at high temperature. Hence, such high concentrations are not preferred either. The sodium ions and potassium ions, that is, the ingredients (B) and (C) in the present invention are derived from water-soluble ingredients or inorganic salts. They are also found in fruit extracts and tea extracts.

Usable sources for sodium ions (B) include readily available sodium salts such as sodium chloride, sodium carbonate, sodium hydrogencarbonate, sodium citrate, sodium phosphate, sodium hydrogenphosphate, sodium tartrate, sodium benzoate and mixtures thereof. Further, those derived from fruit extracts or tea ingredients can also be added. A lower sodium ion concentration is more desired in facilitating the absorption of water under osmotic pressure, although it is important that the sodium ion concentration is at such a level as no water is absorbed into the intestine from the body under osmotic pressure. A sodium ion concentration needed to meet this preferred requirement is preferably lower than sodium ion concentration in the plasma. Taking a look at the possible long-term effect on the color tone at high temperatures, the higher the sodium ion concentration, the higher the degree of color change. From the viewpoint of fat-burning acceleration effect and stability, the content of sodium ions (B) in each of the fat-oxidation promoting, packaged beverage and the like according to the present invention is from 0.001 to 0.5 wt%, preferably from 0.002 to 0.4 wt%, more preferably from 0.003 to 0.2 wt%.

Usable sources for potassium ions (C) include potassium salts such as potassium chloride, potassium carbonate, potassium sulfate, potassium acetate, potassium hydrogencarbonate, potassium citrate, potassium phosphate, potassium hydrogenphosphate, potassium tartrate, potassium sorbate and mixtures thereof. Further, those derived from fruit extracts or tea ingredients are preferred. The potassium ion concentration has been found to have a tendency of giving a greater long-term effect on the color tone than the sodium ion concentration when stored at high temperatures. From the viewpoint of stability, the content of potassium ions (C) in each of the packaged beverage for accelerating fat-burning and the like according to the present invention is from 0.001 to 0.2 wt%, preferably from 0.002 to 0.15 wt%, more preferably from 0.003 to 0.12 wt%.

In addition to sodium ions (B) and potassium ions (C), the packaged beverage for accelerating fat-burning and the like according to the present invention may each contain from 0.001 to 0.5 wt%, preferably from 0.002 to 0.4 wt%, more preferably from 0.003 to 0.3 wt% of chlorine ions. This chlorine ion ingredient can be added in the form of a salt such as sodium chloride or potassium chloride. To a packaged beverage for accelerating fat-burning and the like according to the present invention, a trace of other ions such as calcium, magnesium, zinc and iron ions may be added further. These ions may also be added in the form of salts. The total level of existing ions includes, in addition to the amounts of added ions, the amounts of ions naturally existing in the beverages. When sodium chloride is added, for example, its corresponding amount of sodium ions and its corresponding amount of chloride ions should also be included in the total amounts of respective ions accordingly.

In the packaged beverage for accelerating fat-burning and the like according to the present invention, it is preferred from the standpoint of taste to contain a trace amount of quinic acid or a salt thereof (D) in addition to the above-described ingredients (A) to (C). The content weight ratio of quinic acid or a salt thereof (D) to the non-polymer catechins (A) may be preferably from 0.0001 to 0.5, more preferably from 0.0001 to 0.16, even more preferably from 0.002 to 0.15, even more preferably from 0.002 to 0.1, even more preferably from 0.002 to 0.05. A [(D)/(A)] ratio in this range is preferred, because no strong bitterness, astringency or stypticity is produced, sufficient effect is available for an improvement in the residual perception of the beverage, the sourness of quinic acid is adequate, and the flavor of the beverage is not impaired. Also, such a [(D)/(A)] ratio is preferred, because the beverage is free of a residual perception remaining on the tongue after drinking although such a residual perception is specific to beverages with catechins contained at high concentration and therefore, the beverage is good in the disappearance of an aftertaste. Quinic acid can be added either in the acid form or in a salt form. As an alternative, it can also be added in the form of a composition with quinic acid or a quinic acid salt (D) contained therein. Examples of the quinic acid salt include sodium quinate and potassium quinate.

The mechanisms of the effects of the quinic acid for the reduction of the bitterness and astringency of non-polymer catechins and the improvement in the disappearance of the aftertaste have not been elucidated yet. It is, however, presumed that quinic acid forms weakly-associated units with catechins through hydrogen bonds or the like and the catechins are adsorbed on the gustatory cells to prevent the catechins from contacting the bitterness receptors.

When oxalic acid is contained in the packaged beverage for accelerating fat-burning and the like according to the present invention, the oxalic acid may interact with tea-derived ingredients and added ingredients, which are contained in the beverages (solutions), to form precipitates. In the packaged beverage for accelerating fat-burning and the like, the content of oxalic acid is preferably 0.06 weight part or lower relative to the non-polymer catechins (A). The content of oxalic acid is more preferably 0.05 weight part or lower, even more preferably 0.04 weight part or lower, even more preferably 0.03 weight part or lower. The content of oxalic acid in this range hardly forms precipitates in the packaged beverage for accelerating fat-burning and the like according to the present invention, and therefore, ispreferred from the standpoint of the appearances of the products.

To make an improvement in the taste, a sweetener (E) can be used in the packaged beverage for accelerating fat-burning and the like according to the present invention. As the sweetener (E), an artificial sweetener, carbohydrate or glycerol can be used. Such a sweetener can be contained preferably at from 0.0001 to 20 wt%, more preferably at from 0. 001 to 15 wt%, even more preferably at from 0. 001 to 10 wt%.

Among such artificial sweeteners, examples of the artificial sweetener include high-sweetness sweeteners such as aspartame, saccharin, cyclamate, acesulfame-K, L-aspartyl-L-phenylalanine lower alkyl ester sweetener, L-aspartyl-D-alanine amide, L-aspartyl-D-serine amide, L-aspartyl-hydroxymethylalkanamide sweetener, L-aspartyl-1-hydroxyethylalkanamide sweetener, sucralose and thaumatin, sugar alcohols such as erythritol, xylitol and trehalose, glycyrrhizin, and synthetic alkoxyaromatic compounds. Stevioside and other natural-source sweeteners are also usable.

As a carbohydrate-based sweetener, a soluble carbohydrate is used. A soluble carbohydrate has roles as a sweetener and also, as an energy source. Upon selecting a carbohydrate to be used in the beverage, it is important as a selection standard to assure a sufficient gastric emptying rate and intestinal absorption rate. The soluble carbohydrate can be a mixture of glucose and fructose, or a carbohydrate hydrolyzable into glucose and fructose or capable of forming glucose and fructose in the digestive tract. The term "carbohydrate" as used herein includes monosaccharides, oligosaccharides, conjugated polysaccharides, and mixtures thereof.

Monosaccharide include hexoses and ketohexoses. Examples of the hexoses are aldohexoses such as glucose known as grape sugar. The content of glucose in each of the packaged beverage for accelerating fat-burning and the like according to the present invention may be preferably from 0.0001 to 20 wt%, more preferably from 0.001 to 15 wt%, even more preferably from 0.001 to 10 wt%. Fructose known as "fruit sugar" is a ketohexose. The content of fructose in each of the fat-oxidation promoting packaged beverage and the like according to the present invention may be preferably from 0.0001 to 20 wt%, more preferably from 0.001 to 15 wt%, even more preferably from 0.001 to 10 wt%.

Among sweeteners useful in the packaged beverage for accelerating fat-burning and the like according to the present invention, illustrative oligosaccharides include carbohydrates capable of forming these two species of monosaccharides in the body (i.e., sucrose, maltodextrin, corn syrup, and fructose-rich corn syrup). Of these oligosaccharides, those of an important type are disaccharides. An illustrative disaccharide is sucrose known as cane sugar or beet sugar. The content of sucrose in each of the packaged beverage for accelerating fat-burning and the like according to the present invention may be preferably from 0.001 to 20 wt%, more preferably from 0. 001 to 15 wt%, even more preferably from 0.001 to 10 wt%. It is to be noted that the content of a carbohydrate in each of the packaged beverage for accelerating fat-burning and the like according to the present invention can be indicated as an amount of glucose and an amount of fructose. Namely, the carbohydrate can be hydrolyzed and then, the contents of glucose and fructose can be measured. In the case of an oligosaccharide, the structure of which is known beforehand like sucrose, on the other hand, it is only necessary to convert the amount of the oligosaccharide into its corresponding glucose and fructose amounts.

Preferred examples of the conjugated polysaccharides usable as sweeteners in the present invention are maltodextrins. A maltodextrin is a conjugated polysaccharide the length of which is composed of several glucose units. They are, for example, polysaccharides obtained by the hydrolysis of corn starch. The dextrose equivalent of a maltodextrin is an index of a degree of hydrolysis of a starch polymer.

Carbohydrate-based sweeteners preferred in the present invention are each composed of a combination of fructose and glucose which serve as an energy source capable of supplying necessary calories. As sucrose is completely hydrolyzed into fructose and glucose in the digestive tract, sucrose can be used as a supply source for fructose and glucose. These saccharides are energy foods which can be completely used by body cells. The total content of carbohydrates usable in each of the packaged beverage for accelerating fat-burning and the like according to the present invention is from 0.0001 to 20 wt% based on the whole weight. The total carbohydrate content includes not only those naturally contained in a fruit extract or tea extract but also one or more carbohydrates added. Carbohydrate derivatives, polyhydric alcohols, for example, glycerols, and artificial sweeteners and the like may also be used in the present invention such that they supply sweetener sources and are readily absorbed and distributed throughout the body to supply energy. In the packaged beverage for accelerating fat-burning and the like according to the present invention, glycerols can be used at from 0.1 to 15 wt%, preferably from 0.1 to 10 wt%.

The packaged beverage for accelerating fat-burning and the like according to the present invention can each be obtained by adding a green tea extract and further, other necessary ingredients such as sodium ions and potassium ions.

The term "green tea extract" as used herein can embrace therein one obtained by further purifying a concentrate of an extract of tea leaves in hot water or a water-soluble organic solvent or one obtained by directly purifying the extract. Further, concentrates of green tea extracts, such as commercially-available "POLYPHENON™" (Mitsui Norin Co., Ltd.), "TEAFURAN™" (ITO EN, LTD.) and "SUNPHENON™" (Taiyo Kagaku Co., Ltd.), may also be used after adjusting their compositions.

As a purification method of a concentrate of green tea extract, the concentrate of green tea can be purified, for example, by suspending the concentrate in water or a mixed solution of water and an organic solvent, adding an organic solvent to the resultant suspension, removing the resulting precipitate, and then, distilling off the solvent; or by dissolving the concentrate in an organic solvent, adding water or a mixed solution of water and an organic solvent to the resultant solution, removing the resulting precipitate, and then, distilling off the solvent. It is also preferred to dissolve a concentrate of tea extract, said concentrate preferably containing from 20 to 90 wt.% of non-polymer catechins based on a solid content, in a 9/1 to 1/9 mixed solution of an organic solvent and water and then to bring the resulting solution into contact with activated carbon and acid clay or activated clay. In addition to those mentioned above, it is also possible to use one obtained by purification through supercritical extraction or one obtained by having the concentrate of green tea extract adsorbed on an adsorbent resin and eluting it with an ethanol solution.

As the form of the "green tea extract" as used herein, various forms can be mentioned such as a solid, aqueous solution and slurry. For a shorter history of being dried or the like, an aqueous solution or slurry is preferred.

The green tea extract for use in the present invention may be subjected to an adjustment in composition such that the content weight ratio [(D)/(A)] of quinic acid or a salt thereof (D) to the non-polymer catechins (A) is preferably from 0.0001 to 0.16, more preferably from 0.002 to 0.15, even more preferably from 0.002 to 0.1, even more preferably from 0.002 to 0.05.

The concentration of non-polymer catechins in the green tea extract for use in the present invention can be preferably from 20 to 90 wt%, more preferably from 20 to 87 wt%, even more preferably from 23 to 85 wt%, even more preferably from 25 to 82 wt%.

If the concentration of non-polymer catechins in a green tea extract is lower than 20 wt%, a purified product itself of a green tea extract should be added at a higher concentration to a beverage. If the concentration of non-polymer catechins in a green tea extract exceeds 90 wt%, on the other hand, there is a tendency that trace components and the like other than total polyphenols - which are represented by free amino acids, exist in the green tea extract and serve to improve the flavor - are excluded.

The percentage of gallate, which is a generic term and consists of catechin gallate, epicatechin gallate, gallocatechin gallate and epigallocatechin, based on all non-polymer catechins in the green tea extract for use in the present invention can preferably be from 35 to 100 wt% from the standpoint of the effectiveness of physiological effects of the non-polymer catechins. From the standpoint of the readiness in adjusting the taste, the percentage of gallate may be more preferably from 35 to 98 wt%, even more preferably from 35 to 95 wt%.

The addition of a bitterness suppressor to the packaged beverage for accelerating fat-burning and the like according to the present invention makes them more easily drinkable and therefore, is preferred. As the bitterness suppressor to be used, a cyclodextrin is preferred. As the cyclodextrin, an α-, β- or γ-cyclodextrin or a branched α-, β- or γ-cyclodextrin can be used. In the beverage, a cyclodextrin may be contained at a concentration of from 0.005 to 0.5 wt%, preferably from 0.01 to 0.3 wt%. To the packaged beverage for accelerating fat-burning and the like according to the present invention, it is possible to add, in combination with ingredients derived from tea and as ingredient or ingredients which can be added from the standpoint of formulation, either singly or in combination additives such as antioxidants, flavorings, various esters, organic acids, organic acid salts, inorganic acids, inorganic acid salts, inorganic salts, colorants, emulsifiers, preservatives, seasoning agents, sweeteners, sour seasonings, gums, emulsifiers, oils, vitamins, amino acids, fruit extracts, vegetable extracts, flower honey extracts, pH regulators and quality stabilizers.

A flavoring and/or a fruit extract can be added to make an improvement in taste. In general, fruit extract is called "fruit juice", while flavoring is called "flavor". Natural or synthetic flavorings and fruit extracts can be used in the present invention. They can be selected from fruit juices, fruit flavors, plant flavors, or mixtures thereof. Of these, a combination of a tea flavor, preferably a green tea or black tea flavor in combination with a fruit juice has a preferred taste. Preferred fruit extracts include juices of apple, pear, lemon, lime, mandarin, grapefruit, cranberry, orange, strawberry, grape, kiwi, pineapple, passion fruit, mango, guava, raspberry and cherry. More preferred are citrus juices, with grapefruit, orange, lemon, lime and mandarin juices, mango juice, passion fruit juice and guava juice, and mixtures thereof being even more preferred. A fruit extract may be contained preferably at from 0.001 to 20 wt%, more preferably at from 0. 002 to 10 wt% in the fat-oxidation promoting packaged beverage and the like according to the present invention.

One or more of fruit flavors, plant flavors, tea flavors or a mixture thereof can be used as a flavoring. Preferred natural flavors are jasmine, chamomile, rose, peppermint, <u>Crataegus cuneata</u>, chrysanthemum, water caltrop, sugar cane, bracket fungus of the genus Formes (<u>Formes</u> <u>japonicus</u>), bamboo shoot, and the like. Preferred flavorings are citrus flavors including orange flavor, lemon flavor, lime flavor and grape fruit flavor. As other fruit flavors, apple flavor, grape flavor, raspberry flavor, cranberry flavor, cherry flavor, pineapple flavor and the like can be used. The term "flavoring" as used herein can also include blends of various flavors, for example, a blend of lemon and lime flavors and blends of citrus flavors and selected spices (typically, flavors for cola and other soft drinks). As flavorings consisting of hydrophobic concentrates or extracts, synthetic flavor esters, alcohols, aldehydes, terpenes, sesquiterpenes and the like can be added. Such a flavoring may be contained preferably at from 0.0001 to 5 wt%, more preferably at from 0.001 to 3 wt% in the fat-oxidation promoting packaged beverage and the like according to the present invention.

The packaged beverage for accelerating fat-burning and the like according to the present invention may also contain a sour seasoning. The sour seasoning can be used to maintain the pH of each of the packaged beverage for accelerating fat-burning and the like according to the present invention within a range of from 2 to 7. Acids can be used either in non-dissociated forms or in the form of their sodium or potassium salts. As preferred acids, edible organic acids and inorganic acids can be mentioned including citric acid, malic acid, fumaricacid, adipicacid, gluconic acid, tartaric acid, ascorbic acid, aceticacid, phosphoricacid, and mixtures thereof. More preferred acids are citric acid and malic acid. These sour seasonings are also useful as antioxidants which stabilize beverage ingredients. Examples of other antioxidants include ascorbic acid and plant extracts.

In the packaged beverage for accelerating fat-burning and the like according to the present invention, one or more vitamins can be incorporated further. Preferred vitamins include vitamin A, vitamin C, and vitamin E. Other vitamins such as vitamin D and vitamin B may also be used. One or more minerals can also be used in the beverages according to the present invention. Preferred minerals include calcium, chromium, copper, fluorine, iodine, iron, magnesium, manganese, phosphorus, selenium, silicon, molybdenum, and zinc. More preferred minerals are magnesium, phosphorus, and iron.

The pH of each of the packaged beverage for accelerating fat-burning and the like according to the present invention is from 2 to 7. A pH lower than 2 provides the beverage and the like with a strong sour taste and pungent smell, so that the beverage and the like are not suited for drinking. A pH higher than 7, on the other hand, makes it impossible to provide a balanced flavor, leading to a deterioration in taste. Moreover, the stability is also deteriorated. A preferred pH is from 2 to 6, with a pH of from 2 to 4.5 being more preferred.

The packaged beverage for accelerating fat-burning and the like according to the present invention can be preferably in the form of drinks, more preferably in the form of packaged drinks, although they can be in any form insofar as they can be orally ingested.

As target consumers of the packaged beverage for accelerating fat-burning and the like according to the present inventions, it is possible to label or otherwise indicate as "FOR CONSUMERS WHO ARE CONCERNED WITH BODY FAT". To indicate their function as products for fat oxidation, it is possible to describe that they can facilitate the use of fat or body fat.

When the packaged beverage for accelerating fat-burning and the like according to the present invention are combined with an exercise load, their fat-burning acceleration effect is pronouncedly enhanced. In other words, the exercising fat metabolism is enhanced further by the ingestion of the beverage. Further, the combination of the ingestion of the beverage with an exercise habit increases the body-fat reducing effect. Furthermore, the beverage is effective in facilitating the use of fat as energy, especially as exercising energy. When the intensity of exercise is used as an index of the use of energy during exercise, the exercise intensity may be from about 30 to 100% HRmax, preferably from about 30 to 80% HRmax, more preferably from about 30 to 60% HRmax (maximum heart rate). The packaged beverage for accelerating fat-burning according to the present invention can, therefore, be labeled or otherwise indicated as "THIS BEVERAGE IS EFFECTIVE WHEN COMBINED WITH EXERCISE HABIT" or "THIS BEVERAGE FACILITATES THE USE OF FAT AS EXCERCISING ENERGY".

When the beverage according to the present invention is continuously ingested, the burning of fat is accelerated and the energy-consumption is boosted. As a result, the maximum oxygen uptake as an index of the enhancement of exercise endurance increases pronouncedly. The beverage according to the present invention can, therefore, be labeled as "THIS BEVERAGE ACCELERATES FAT-BURNING, BOOSTS ENERGY-CONSUMPTION, ENHANCES EXERCISE ENDURANCE, AND MAKES RESISTANT TO BECOME TIRED EVEN WHEN EXERCISING".

The packaged beverage for accelerating fat-burning and the like according to the present invention can be ingested preferably as much as 300 mg or more in terms of non-polymer catechins per adult and day, with 450 mg or more being more preferred and 500 mg or more being even more preferred.

Packaged beverages according to the present invention include, for example, carbonated beverages as soft drinks, beverages with fruit extracts, juices with vegetable extracts, near waters, sports drinks, isotonic drinks, diet drinks, and the like. Further, they can also be used as specific health foods, functional health foods, or beauty foods.

Similar to general beverages, packaged beverages according to the present invention can each be provided in an ordinary form packaged in a molded package made of polyethylene terephthalate as a principal component (a so-called PET bottle), a metal can, a paper container combined with metal foils or plastic films, or a bottle. The term "packaged beverage" as used herein means one that can be consumed without dilution.

The packaged beverage according to the present invention can be produced, for example, by filling the beverage in a container such as a metal can and, when heat sterilization is feasible, conducting heat sterilization under sterilization conditions as prescribed in the Food Sanitation Act (Japan). For those which cannot be subjected to retort sterilization like PET bottles or paper containers, a process is adopted such that the beverage is sterilized beforehand under similar sterilization conditions as those described above, for example, is sterilized at a high temperature for a short time by a plate-type heat exchanger, is cooled to a prescribed temperature, and is then filled in a container. Under aseptic conditions, additional ingredients may be added to and filled in a beverage-filled container. It is also possible to conduct an operation such that subsequent to heat sterilization under acidic conditions, the pH of the beverage is caused to arise back to neutral under aseptic conditions or that subsequent to heat sterilization under neutral conditions, the pH of the tea beverage is caused to drop back to the acidic side under aseptic conditions.

**Examples**

Measurement of catechins

A high-performance liquid chromatograph (model: "SCL-10AVP", trade name) manufactured by Shimadzu Corporation was used. The chromatograph was fitted with an LC column packed with octadecyl-introduced silica gel, "L-Column, TM ODS" (trade name; 4.6 mm in diameter $\times$ 250 mm in length; product of Chemicals Evaluation and Research Institute, Japan). A packaged beverage, which had been filtered through a filter (0.8 $\mu$m) and then diluted with distilled water, was subjected to chromatography at a column temperature of 35°C by gradient elution. A 0.1 mol/L solution of acetic acid in distilled water and a 0.1 mol/L solution of acetic acid in acetonitrile were used as mobile phase solution A and mobile phase solution B, respectively. The measurement was conducted under the conditions of 20 $\mu$L injected sample quantity and 280 nm UV detector wavelength.

Quantitation of sodium ions

Atomic fluorescence spectroscopy (extraction with hydrochloric acid)

Each sample (5 g) was placed in 10% hydrochloric acid (to provide a 1% HCl solution when dissolved to a predetermined volume). With deionized water, the resulting solution was then brought to the predetermined volume, and its absorbance was measured.
Wavelength: 589.6 nm
Flame: acetylene-air

Quantitation of potassium ions

Atomic fluorescence spectroscopy (extraction with hydrochloric acid)

Each sample (5 g) was placed in 10% hydrochloric acid (to provide a 1% HCl solution when dissolved to a predetermined volume). With deionized water, the resulting solution was then brought to the predetermined volume, and its absorbance was measured.

Measurement of quinic acid

The Japan Food Research Laboratories method relying upon HPLC

Each sample (2 g) was filtered subsequent to ultrasonication, and was then measured by high-performance liquid chromatography.
Model: "LC-10AD" (trade name, Shimadzu Corporation) Detector: UV-visible spectrophotometer, "SPD-6AV" (trade name, Shimadzu Corporation)
Column: "TSKGEL OApak" (trade name, 7. 8 mm in diameter $\times$ 300 mm in length, TOSOH CORPORATION)
Column temperature: 40°C

Mobile phase: 0.75 mmol/L sulfuric acid
Reaction solution: 15 mmol/L solution of disodium hydrogenphosphate, which contained 0.2 mmol/L bromothymol blue.
Measurement wavelength: 445 nm
Flow rates: Mobile phase - 0.8 mL/min Reaction solution - 0.8 mL/min

Measurement of oxalic acid

An ion chromatograph (model: DXAQ1110, manufactured by Japan Dionex Co., Ltd.) was fitted with a column, "IonPac AS4A-SC" (trade name, 4 mm in diameter $\times$ 250 mm in length) and was connected to a suppressor, "ASRS-ULTRA" (trade name; manufactured by Dionex Corporation). Measurement of oxalic acid was performed in the recycle mode. As mobile phases, 1.8 mmol/L $Na_2CO_3$ and 1.7 mmol/L $NaHCO_3$ were fed at 1.0 mL/min. The injected sample quantity was set at 25 μL. An electrical conductivity detector was used as a detector.

Example 1 & Comparative Example 1

The packaged beverage of each example or comparative example was produced by mixing the corresponding ingredients shown in Table 1 and then conducting predetermined post-processing.
In accordance with the sports drink formulation shown in Table 1, the individual ingredients (weight parts) were combined together, and then messed up with deionized water so that a mixed solution was prepared. Based on the Food Sanitation Act (Japan), sterilization and hot-pack filling were conducted to produce a packaged beverage. Ingredient data of the beverage are also shown.
Table 1

| Ingredients | Example 1 | Comp. Ex. 1 |
|---|---|---|
| Non-polymer catechins | 114 mg/100 mL | 0 |
| Sweetener | 1.20% | 1.20% |
| Sour seasoning | 0.24% | 0.24% |
| Na | 40 mg/100 mL | 40 mg/100 mL |
| K | 9 mg/100 mL | 9 mg/100 mL |
| Flavor | 0.30% | 0.30% |
| Vitamin C | 0.03% | 0.03% |
| Deionized water | Balance | Balance |
| Total volume | 500 mL | 500 mL |
| pH | 3.5 | 3.5 |
| Calories | 4 kcal/100 mL | 4 kcal/100 mL |

Example 2

(1) Method

A test was conducted on nineteen healthy men, aged at 37.6 on average, (excluding those unfit for exercise and those suffering from any disease possibly associated with an energy metabolism disorder). They were requested to ingest the beverage of Example 1 or Comparative Example 1, which is described in Table 1, as much as one bottle (500 mL) per day for 12 weeks, and were then assessed in athletic capacity (maximum oxygen uptake; exercising, fat and carbohydrate burning).
For the assessment of the athletic capacity of each man, he exercised, in which the load was increased stepwise, on a bicycle ergometer ("AEROBIKE 75XL", trade name; manufactured by Combi Corporation) while measuring his breath composition and heart rate Measurements were started from a resting time, the load was increased at a rate of 15 watt/min, and the exercise was continued up to about 60% HRmax. His oxygen uptake, carbon dioxide excretion and heart rate were measured to calculate hismaximumoxygenuptake ($VO_2$max) and exercising? fat burning.
The exercise loads applied in that test were equivalent to about 20 to 60 cal/kg·min when converted to energy consumption. In terms of heart rate, they were equivalent to about 65 to 100 beats/min. In terms of exercise intensity expressed using the heart rate as an index, they were equivalent to about 30 to 60% HRmax. In terms of the rating of perceived exertion (RPE, as assessed on the Borg scale), they were equivalent to 6 (rest) to 11 (fairly light). In terms of METs, they were equivalent to about 1 to 3.5 METs. When 3.5 METs, the maximum value in METs in that test, are converted into everyday activities or exercises, this maximum MET value corresponds to gardening or weed pulling (3 to 4 METs), playing ball (2 to 4 METs), cycling (3 to 8 METs), and hiking (3 to 7 METs). The range of the exercise loads in the above test, therefore, covers from a resting state to exercise intensities which are certainly experienced in everyday life.

The term "maximum oxygen uptake ($VO_2$max) " as used herein means the maximum volume of oxygen (mL/min·kg) that can be uptaken per kg of body weight. From a regression line between heart rate and oxygen uptake when an exercise in which the load was increased stepwise was performed, an oxygen uptake corresponding to the maximum heart rate (220 - age) was calculated as $VO_2$max. This method is called "the submaximal exercise load test", and is a general measuring method of $VO_2$max.
$VO_2$max is a value that indicates aerobic energy availability, and is used as an index of whole-body endurance in the field of exercise physiology. $VO_2$max is considered to negatively correlate with the morbidity rate of life-style related diseases, and its target values to be maintained as broken down by age are prescribed. Owing to the existence of a positive correlation between $VO_2$max and skeletal muscle mass, $VO_2$max is also regarded as important in recent years as an index of muscle mass to be maintained for the purpose of preventing sarcopenia or osteopenia (as a result of aging, the protein-synthesizing ability of cells decreases, resulting in weakened muscles and bones). It is also an index of the enhancement in exercise endurance as a result of an acceleration in fat-burning and an enhancement in energy-consumption.

For a respiratory analysis, a respiratory metabolic analysis system ("Vmax29", trade name; manufactured by Sensor Medics Corporation, Yorba Linda, California, U.S.A.) was used. By introducing both breath and air, oxygen uptake, carbon dioxide excretion, respiratory quotient and energy expenditure were calculated based on the differences between the concentrations of oxygen and carbon dioxide in air and the concentrations of oxygen and carbon dioxide in breath. It is to be noted that the calculations of energy expenditure, fat-derived energy expenditure and carbohydrate-derived energy expenditure were conducted in accordance with the following formula:

Definition:

CV: energy per liter of oxygen (Kcal/L)
RQ: respiratory quotient oxygen/carbon dioxide (mol/mol)
$Q_2$ : oxygen expenditure (L/min)

Correlation formula between CV and RQ:

$$CV = 3.815 + 1.232 + RQ$$

Correlation formula between energy expenditure and CV and $O_2$ :

One day consists of 1,440 minutes. Hence,

$$Energy\ expenditure\ (Kcal/day) = CV \times O_2 \times 1,440$$

Further, fat-derived energy expenditure which reflects the oxidation of fat was determined by the method of Zuntz et al.
The exercising fat burning (cal/min · kg) was determined by multiplying the total energy consumption with the fat/carbohydrate burning ratio obtained from the respiratory quotient. As an inter-group difference was observed in body fat percentage, each assessment was performed based on a value per free fat mass. Under a low load of fat burning (cal/min · kg) exercise, fat-derived energy is primarily used, and the fat burning increases with the exercise load. Under a high load of exercise, carbohydrate-derived energy is also used in addition to fat-derived energy. In this case, carbohydrates are anaerobically metabolized to produce lactic acid. Since RQ increases by a rise in breath $CO_2$ concentration

as a result of the production of lactic acid, a decrease in the metabolic rate of fat energy is observed in appearance. Actually, however, the metabolic rate of fat energy is considered to remain unchanged.

(2) Results

The maximum oxygen uptake ($VO_2$max) remained substantially unchanged ($0.1\pm2.9$ mL/min · kg) in the Comparative Example 1 beverage ingestion group even week 12 as shown in Table 2, whereas it significantly increased to $5.5\pm1.0$ mL/min · kg in the Example 1 beverage ingestion group. In a taste assessment of the beverages by consecutive two-week drinking, the taste was good.

Table 2

|  | $VO_2$max (mL/min · kg) | | |
|---|---|---|---|
|  | Week 0 | Week 12 | $\Delta$ (week 12 - week 0) |
| 0 mg (n=9) | $35.2\pm2.0$ | $35.4\pm2.7$ | $0.1\pm2.9$ |
| 540 mg (n=10) | $33.1\pm2.3$ | $38.7\pm2.4$* | $5.5\pm1.0$# |
| Mean$\pm$SEM, * : vs OW (paired t-test, p<0.05), #:vs 0 mg (t-test, p<0.083) | | | |

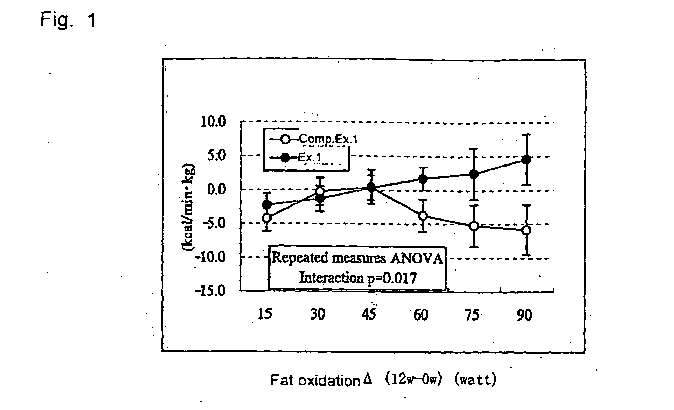

As shown in Table 3, Table 4 and FIG. 1, it was found that the exercising fat burning (cal/min · kg) remained substantially unchanged despite the exercise load in the Comparative Example 1 beverage ingestion group and also that the exercising fat burning (cal/min · kg) in the Example 1 beverage ingestion group was not observed to differ from that of the Comparative Example 1 beverage ingestion group in the range of from the resting time to the very low intensities (about 15 to 30 watt) but was greater with a statistical significance than that of the Comparative Example 1 beverage ingestion group in the increased exercise range of from 45 to 90 watt.

Table 3

|  |  | Group | Exercise intensity (Watt) | | | | | | P value | | |
|---|---|---|---|---|---|---|---|---|---|---|---|
|  |  |  | 15 | 30 | 45 | 60 | 75 | 90 | Ex. 1 | Exercise load | Example x exercise load |
| Fat expenditure (cal/kg·min) | Week 0 | Comp. Ex. 1 | 17.9±1.6 | 18.7±1.7 | 18.3±1.8 | 24.8±1.7 | 34.4±4.6 | 36.2±4.1 | 0.729 | <0.001 | 0.399 |
|  |  | Ex. 1 | 15.9±1.6 | 21.8±1.8 | 19.6±2.0 | 23.0±2.1 | 31.3±2.8 | 32.1±4.7 |  |  |  |
|  | Week 12 | Comp. Ex. 1 | 13.6±2.1 | 18.4±1.8 | 18.8±2.3 | 21.1±2.9 | 29.2±3.8 | 30.4±2.8 | 0.276 | <0.001 | 0.310 |
|  |  | Ex. 1 | 13.6±1.1 | 20.5±1.3 | 20.0±1.4 | 24.8±1.9 | 33.8±2.2 | 36.7±3.1 |  |  |  |
|  | Δ (week 12 - week 0) | Comp. Ex. 1 | -4.2±1.8 | -0.3±1.9 | 0.5±1.8 | -3.7±1.7 | -5.3±3.8 | -5.8±3.7 | 0.175 | 0.528 | 0.017 |
|  |  | Ex. 1 | -2.3±1.9 | -1.3±2.0 | 0.4±2.5 | 1.8±2.4 | 2.5±3.1 | 4.6±3.7 |  |  |  |

Means±SEM, n=9 or 10.

Table 4

|  | Group | Week 0 | Week 12 | $\Delta$ (week 12 - week 0) |
|---|---|---|---|---|
| REE (cal/kg · min) | Comp. Ex. 1 | $24.9\pm1.6$ | $29.4\pm1.4$ | $-0.5\pm1.7$ |
|  | Ex. 1 | $26.9\pm0.9$ | $27.9\pm1.6$ | $1.0\pm1.9$ |
| RQ (-) | Comp. Ex. 1 | 0.781+0.028 | 0.784+0.030 | 0.003+0.015 |
|  | Ex. 1 | 0.811+0.031 | 0.799+0.015 | -0.012+0.028 |

(continued)

|  | Group | Week 0 | Week 12 | Δ (week 12 - week 0) |
|---|---|---|---|---|
| CHO (cal/kg · min) | Comp. Ex. 1 | 7.1±2.9 | 6.7±3.0 | -0.4±1.2 |
| | Ex. 1 | 9.1±2.7 | 8.7±1.3 | -0.4±2.7 |
| FAT (cal/kg · min) | Comp. Ex. 1 | 17.9±2.3 | 17.5±2.8 | -0.4±2.6 |
| | Ex. 1 | 17.9±3.3 | 19.3±2.1 | 1.3±2.9 |
| Mean±SEM, n=9 or 10, REE: resting energy expenditure, RQ: respiratory quotient, CHO: carbohydrates | | | | |

Example 3

(1) Method

A test was conducted on somewhat obese healthymen. They were requested to ingest the beverage of Example 1 or Comparative Example 1 for 12 weeks, and their body fat masses and abdominal fat areas were then measured.
The Comparative Example 1 beverage ingestion group and the Example 1 beverage ingestion group were each divided into two subgroups depending on the body activity level estimated from the questioning about exercise habit and the counting of walking steps. The subgroups so divided were of the following characteristics.
1) Low exercise habit group: Throughout the test period, exercise was performed once a week or less, and 9,000 steps or less were walked in a week.
2) Medium exercise habit group: A group not falling in the group 1). As those having an intense exercise habit had been excluded beforehand at a screening stage in this test, this group was classified as a medium exercise habit group.

By the above sub-grouping, four subgroups were obtained in total, including a low exercise habit subgroup (n=13) and medium exercise habit group (n=16) with no ingestion of the beverage according to the present invention, and a low exercise habit subgroup (n=28) and medium exercise habit group (n=25) with ingestion of the beverage according to the present invention.
In each subgroup, during the test period, meals and exercise habit were taken and performed as usual, and the test beverage was ingested as much as one bottle (500 mL, 540 mg in terms of catechins) per day.
The body fat percentage was determined by a bioelectrical impedance body-fat meter ("TF-780", trade mark; Tanita Corporation), and from an image obtained by a transverse CT scan at the level of the L4/L5 disc, the abdominal total fat area (TFA), abdominal visceral fat area (VFA) and abdominal subcutaneous fat area (SFA) were determined in accordance with the method reported by Tokunaga et al. while employing the visceral fat measurement PC software, "FAT SCAN VER. 2" (trade name; N2 System Corporation. Using "ASTEION" (trademark; Toshiba Medical Systems Corporation, Ohtawara, Tochigi, Japan) as an X-ray CT scanner, X-ray scans were conducted at a tube voltage of 120 kVp and a tube current of 200 mAs. The images were analyzed using a window level of 0 and a window width of 1,000.

(2) Results

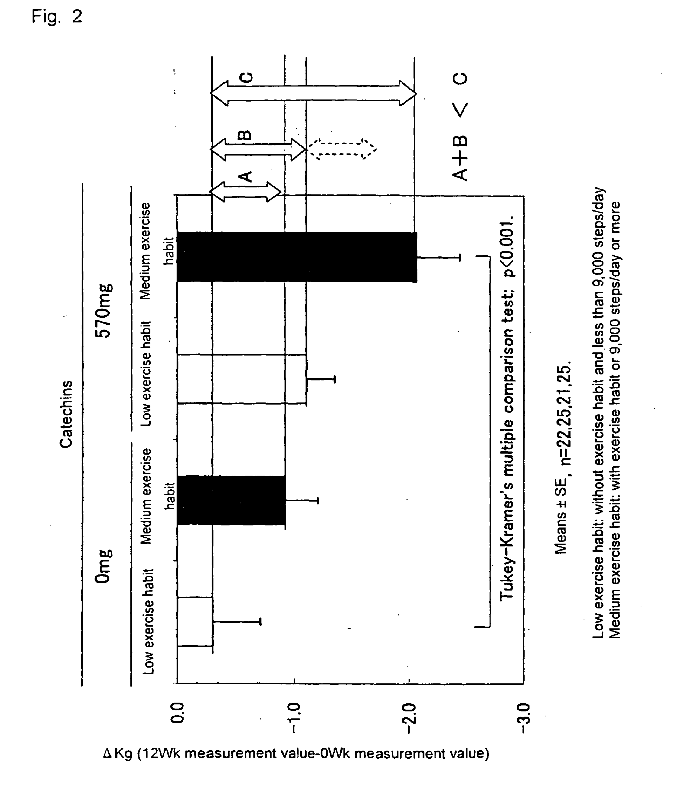

The results are shown in Table 5. Differences in body weight between week 0 and week 12 are shown, respectively, in FIG. 2.
As clearly envisaged from Table 5 and FIG. 2, it has been found that a combination of the beverage according to the present invention and an exercise habit leads to a significant reduction in body weight.
# EP 1 813 156 B1

Table 5

|  | Exercise habit | n |  | Body weight (kg) Week 0 | Δ(Wk12-Wk0) | BMI (kg/m²) Week 0 | Δ(Wk12-Wk0) | Body fat percentage (%) Week 0 | Δ(Wk12-Wk0) | Waist (cm) Week 0 | Δ(Wk12-Wk0) |
|---|---|---|---|---|---|---|---|---|---|---|---|
| Comp.Ex.1 | Without | 22 | Mean | 76.25 | -0.30 | 25.96 | -0.10 | 26.40 | -0.67 | 88.14 | 0.07 |
| Comp.Ex.1 | With | 25 | Mean | 71.95 | -0.92 | 24.43 | -0.33 | 24.18 | -1.42 | 85.51 | -0.75 |
| Ex.1 | Without | 21 | Mean | 74.90 | -1.11 | 26.07 | -0.39 | 26.56 | -2.53 | 88.46 | -0.48 |
| Ex.1 | With | 25 | Mean | 73.87 | -2.06 | 25.03 | -0.69 | 24.42 | -2.89 | 85.02 | -1.11 |
|  |  |  | SE | 2.11 | 0.41 | 0.61 | 0.14 | 1.13 | 0.37 | 1.50 | 0.47 |
|  |  |  | SE | 1.32 | 0.29 | 0.37 | 0.10 | 0.72 | 0.33 | 0.82 | 0.38 |
|  |  |  | SE | 2.20 | 0.25 | 0.61 | 0.09 | 0.97 | 0.57 | 1.49 | 0.46 |
|  |  |  | SE | 1.44 | 0.37 | 0.47 | 0.13 | 0.69 | 0.30 | 1.13 | 0.50 |

|  | Exercise habit | n |  | Hip (cm) Week 0 | Δ(Wk12-Wk0) | TFA (cm²) Week 0 | Δ(Wk12-Wk0) | VFA (cm²) Week 0 | Δ(Wk12-Wk0) | SFA (cm²) Week 0 | Δ(Wk12-Wk0) |
|---|---|---|---|---|---|---|---|---|---|---|---|
| Comp.Ex.1 | Without | 22 | Mean | 95.02 | 0.53 | 248.19 | -0.19 | 78.76 | 1.60 | 169.42 | -1.78 |
| Comp.Ex.1 | With | 25 | Mean | 93.19 | 0.14 | 206.35 | -0.80 | 68.59 | 2.85 | 137.76 | -3.65 |
| Ex.1 | Without | 21 | Mean | 94.54 | -0.58 | 268.19 | -23.63 | 94.34 | -9.96 | 173.85 | -13.68 |
| Ex.1 | With | 25 | Mean | 94.42 | -1.22 | 214.17 | -28.63 | 64.96 | -9.92 | 149.21 | -18.71 |
|  |  |  | SE | 1.08 | 0.28 | 17.47 | 6.03 | 7.18 | 3.06 | 14.99 | 3.54 |
|  |  |  | SE | 0.64 | 0.24 | 13.11 | 5.77 | 5.95 | 3.06 | 8.68 | 3.42 |
|  |  |  | SE | 1.12 | 0.33 | 18.10 | 6.15 | 8.35 | 3.36 | 13.19 | 3.62 |
|  |  |  | SE | 0.82 | 0.34 | 11.68 | 4.91 | 4.86 | 2.77 | 10.41 | 3.36 |

Low exercise habit: without exercise habit and less than 9,000 steps/day
Medium exercise habit: with exercise habit or 9,000 steps/day or more
Tukey-Kramer's multiple comparison test: *, $p<0.05$; **, $p<0.01$.

## Claims

1. Non-therapeutic use of a packaged beverage, which contains a green tea extract added therein and has a pH of from 2 to 7, and further contains the following ingredients (A) to (C):

   (A) from 0.01 to 1.0 wt% of non-polymer catechins,
   (B) from 0.001 to 0.5 wt% of sodium ions, and
   (C) from 0.001 to 0.2 wt% of potassium ions,

   for accelerating fat-burning in combination with exercise.

2. Non-therapeutic use of a packaged beverage as defined in claim 1 for boosting energy-consumption in combination with exercise.

3. Non-therapeutic use of a packaged beverage as defined in claim 1 for enhancing exercise-endurance.

4. Non-therapeutic use of a packaged beverage as defined in claim 1 for preventing exercise-induced fatigue.

5. The non-therapeutic use according to any of the claims 1-4, wherein the beverage is to be ingested in conjunction with an exercise load having an exercise intensity of from 30 to 100% HRmax.

6. A non-therapeutic method for accelerating fat-burning in combination with exercise, which comprises consuming a packaged beverage as defined in claim 1 in combination with exercise.

7. A non-therapeutic method for boosting energy-consumption in combination with exercise, which comprises consuming a packaged beverage as defined in claim 1 in combination with exercise.

8. A non-therapeutic method for enhancing exercise-endurance, which comprises consuming a packaged beverage as defined in claim 1 in combination with exercise.

9. A non-therapeutic method for preventing exercise-induced fatigue, which comprises consuming a packaged beverage as defined in claim 1 in combination with exercise.

10. The non-therapeutic method according to any of the claims 6-9, wherein the beverage is ingested in conjunction with an exercise load having an exercise intensity of from 30 to 100% HRmax (maximum heart rate).

11. A packaged beverage, which contains a green tea extract added therein and has a pH of from 2 to 7, and further contains the following ingredients (A) to (C):

> (A) from 0.01 to 1.0 wt% of non-polymer catechins,
> (B) from 0.001 to 0.5 wt% of sodium ions, and
> (C) from 0.001 to 0.2 wt% of potassium ions,

for use in a method of accelerating fat-burning in combination with exercise for the reduction of body fat in the prevention of obesity.

**Patentansprüche**

1. Nicht-therapeutische Verwendung eines verpackten Getränks, das ein darin hinzugefügtes Grüntee-Extrakt enthält und einen pH von 2 bis 7 aufweist, und weiterhin die folgenden Inhaltsstoffe (A) bis (C) enthält:

> (A) von 0,01 bis 1,0 Gew.% nicht-polymere Catechine,
> (B) von 0,001 bis 0,5 Gew.% Natriumionen, und
> (C) von 0,001 bis 0,2 Gew.% Kaliumionen,

zum Beschleunigen der Fettverbrennung in Kombination mit Bewegungsübungen.

2. Nicht-therapeutische Verwendung eines verpackten Getränks wie in Anspruch 1 definiert zum Verstärken des Energieverbrauchs in Kombination mit Bewegungsübungen.

3. Nicht-therapeutische Verwendung eines verpackten Getränks wie in Anspruch 1 definiert zum Verbessern der Übungsausdauer.

4. Nicht-therapeutische Verwendung eines verpackten Getränks wie in Anspruch 1 definiert zum Verhindern von durch Übung verursachter Ermüdung.

5. Nicht-therapeutische Verwendung gemäß irgendeinem der Ansprüche 1 bis 4, worin das Getränkt für die Einnahme in Verbindung mit einer Übungslast vorgesehen ist, die eine Übungsintensität von 30 bis 100 % HRmax hat.

6. Nicht-therapeutische Verfahren zum Beschleunigen der Fettverbrennung in Kombination mit Bewegungsübungen, das das Einnehmen eines verpackten Getränks wie in Anspruch 1 definiert in Kombination mit Bewegungsübungen umfasst.

7. Nicht-therapeutische Verfahren zur Verstärkung des Energieverbrauchs in Kombination mit Bewegungsübungen, das das Einnehmen eines verpackten Getränks wie in Anspruch 1 definiert in Kombination mit Bewegungsübungen umfasst.

8. Nicht-therapeutische Verfahren zum Erhöhen der Übungsausdauer, das das Einnehmen eines verpackten Getränks wie in Anspruch 1 definiert in Kombination mit Bewegungsübungen umfasst.

9. Nicht-therapeutische Verfahren zum Verhindern von durch Übung verursachter Ermüdung, das das Einnehmen eines verpackten Getränks wie in Anspruch 1 definiert in Kombination mit Bewegungsübungen umfasst.

10. Nicht-therapeutische Verfahren gemäß irgendeinem der Ansprüche 6 bis 9, worin das Getränk in Verbindung mit einer Übungslast eingenommen wird, die eine Übungsintensität von 30 bis 100 % HRmax (maximale Herzrate) hat.

11. Verpacktes Getränkt, das ein darin hinzugefügtes Grüntee-Extrakt enthält und einen pH von 2 bis 7 aufweist, und weiterhin die folgenden Inhaltsstoffe (A) bis (C) enthält:

> (A) von 0,01 bis 1,0 Gew.% an nicht-polymere Catechine,

(B) von 0,001 bis 0,5 Gew.% an Natriumionen, und
(C) von 0,001 bis 0,2 Gew.% an Kaliumionen,

zur Verwendung in einem Verfahren zum Beschleunigen der Fettverbrennung in Kombination mit Bewegungsübung zur Verringerung des Körperfetts in der Prävention von Fettleibigkeit.

**Revendications**

1. Utilisation non thérapeutique d'une boisson conditionnée, qui contient un extrait de thé vert ajouté dedans et a un pH de 2 à 7, et contient en outre les ingrédients (A) à (C) suivants :

(A) de 0,01 à 1,0% en poids de catéchines non polymères,
(B) de 0,001 à 0,5% en poids d'ions sodium, et
(C) de 0,001 à 0,2% en poids d'ions potassium,

pour accélérer la combustion des graisses en combinaison avec l'exercice.

2. Utilisation non thérapeutique d'une boisson conditionnée telle que définie dans la revendication 1 pour augmenter la consommation d'énergie en combinaison avec l'exercice.

3. Utilisation non thérapeutique d'une boisson conditionnée telle que définie dans la revendication 1 pour améliorer l'endurance à l'exercice.

4. Utilisation non thérapeutique d'une boisson conditionnée telle que définie dans la revendication 1 pour la prévention de la fatigue induite par l'exercice.

5. Utilisation non thérapeutique selon l'une quelconque des revendications 1 à 4, où la boisson doit être ingérée conjointement avec une charge d'exercice ayant une intensité d'exercice de 30 à 100% HR max.

6. Procédé non thérapeutique pour accélérer la combustion des graisses, en combinaison avec l'exercice, qui comprend la consommation d'une boisson conditionnée telle que définie dans la revendication 1, en combinaison avec l'exercice.

7. Procédé non thérapeutique pour augmenter la consommation d'énergie en combinaison avec l'exercice, qui comprend la consommation d'une boisson conditionnée telle que définie dans la revendication 1 en combinaison avec l'exercice.

8. Procédé non thérapeutique pour améliorer l'endurance à l'exercice, qui comprend la consommation d'une boisson conditionnée telle que définie dans la revendication 1 en combinaison avec l'exercice.

9. Procédé non thérapeutique pour la prévention de la fatigue induite par l'exercice, qui comprend la consommation d'une boisson conditionnée telle que définie dans la revendication 1 en combinaison avec l'exercice.

10. Procédé non thérapeutique selon l'une quelconque des revendications 6 à 9, dans lequel la boisson est ingérée conjointement avec une charge d'exercice ayant une intensité d'exercice de 30 à 100% HR max (fréquence cardiaque maximale).

11. Boisson conditionnée, qui contient un extrait de thé vert ajouté dedans et a un pH de 2 à 7, et contient en outre les ingrédients (A) à (C) suivants :

(A) de 0,01 à 1,0% en poids de catéchines non polymères,
(B) de 0,001 à 0,5% en poids d'ions sodium, et
(C) de 0,001 à 0,2% en poids d'ions potassium,

à utiliser dans un procédé d'accélération de combustion des graisses, en combinaison avec l'exercice pour diminuer les réserves lipidiques de l'organisme dans le but de la prévention de l'obésité.

Fig. 1

Fat oxidation Δ (12w–0w) (watt)

Fig. 2

EP 1 813 156 B1

Catechins

| 0mg | | 570mg | |
|---|---|---|---|
| Low exercise habit | Medium exercise habit | Low exercise habit | Medium exercise habit |

A+B < C

Tukey-Kramer's multiple comparison test; p<0.001.

ΔKg (12Wk measurement value-0Wk measurement value)

Means ± SE, n=22,25,21,25.

Low exercise habit: without exercise habit and less than 9,000 steps/day
Medium exercise habit: with exercise habit or 9,000 steps/day or more

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60156614 A
- JP 3133928 A
- JP 2002326932 A

**Non-patent literature cited in the description**

- **Yoshida et al.** *J. Nutr. Sci. Vitaminol,* 1988, vol. 34 (6), 587-594
- **Yoshioka et al.** *J. Nutr. Sci . Vitaminol,* April 1990, vol. 36 (2), 173-178